(19) Europäisches Patentamt
European Patent Office
Office européen des brevets

(11) EP 3 350 159 B1

(12) **FASCICULE DE BREVET EUROPEEN**

(45) Date de publication et mention de la délivrance du brevet:
**07.08.2019 Bulletin 2019/32**

(21) Numéro de dépôt: **16781837.6**

(22) Date de dépôt: **16.09.2016**

(51) Int Cl.:
***C07C 303/02*** $^{(2006.01)}$ ***C08F 220/56*** $^{(2006.01)}$
***C07C 309/15*** $^{(2006.01)}$

(86) Numéro de dépôt international:
**PCT/FR2016/052359**

(87) Numéro de publication internationale:
**WO 2017/046546 (23.03.2017 Gazette 2017/12)**

(54) **PROCEDE D'OBTENTION DU MONOMERE ACIDE 2-ACRYLAMIDO-2-MÉTHYLPROPANESULPHONIQUE ET POLYMERE COMPRENANT LEDIT MONOMERE**

VERFAHREN ZUR HERSTELLUNG EINES 2-ACRYLAMIDO-2-METHYLPROPANSULFONSÄUREMONOMERS UND POLYMER MIT DIESEM MONOMER

METHOD FOR PRODUCING THE 2-ACRYLAMIDO-2-METHYLPROPANE SULFONIC ACID MONOMER AND POLYMER COMPRISING SAID MONOMER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **18.09.2015 FR 1558796**

(43) Date de publication de la demande:
**25.07.2018 Bulletin 2018/30**

(73) Titulaire: **S.P.C.M. SA**
**42160 Andrézieux Bouthéon (FR)**

(72) Inventeurs:
- **FAVERO, Cédrick**
**42610 Saint Romain Le Puy (FR)**
- **KIEFFER, Johann**
**42210 Hopital Le Grand (FR)**

(74) Mandataire: **Cabinet Laurent & Charras**
**Le Contemporain**
**50 Chemin de la Bruyère**
**69574 Dardilly Cedex (FR)**

(56) Documents cités:
**CN-A- 101 066 940** **CN-A- 102 351 744**
**JP-A- 2010 270 170**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 350 159 B1

## Description

### DOMAINE DE L'INVENTION

Le domaine de l'invention concerne un nouveau procédé d'obtention de l'acide 2-acrylamido-2-méthylpropane sulfonique. Plus précisément, l'invention concerne un procédé permettant d'obtenir l'acide 2-acrylamido-2-méthylpropane sulfonique à partir d'isobutylène ayant une teneur en butène inférieure à 100 ppm et en butadiène inférieure à 100 ppm.

### ETAT ANTERIEUR DE LA TECHNIQUE

L'acide 2-acrylamido-2-méthylpropane sulfonique est largement utilisé comme additif dans les fibres acryliques ou encore comme matière première pour des polymères utilisés en tant que dispersant ou épaississant pour les secteurs variés comme la cosmétique ou encore l'industrie pétrolière.

Les polymères spécifiquement utilisés comme épaississants dans la récupération assistée du pétrole, sont produits par polymérisation de plusieurs monomères dont au moins un monomère est l'acide 2-acrylamido-2-méthylpropane sulfonique. Ces polymères sont en général de très haut poids moléculaires avec un très faible taux d'insolubles dans l'eau.

A titre d'exemple, le document JP2010-270170 décrit l'obtention de polymères de haut poids moléculaire contenant au moins l'acide 2-acrylamido-2-méthylpropane sulfonique et son utilisation dans la récupération assistée du pétrole.

L'acide 2-acrylamido-2-méthylpropane sulfonique est couramment fabriqué par réaction entre l'acrylonitrile, l'acide sulfurique fumant (oleum) et l'isobutylène dans des proportions stoechiométriques. L'acrylonitrile est cependant utilisé en excès par rapport à l'acide sulfurique fumant et l'isobutylène car il joue le rôle de solvant de la réaction.

L'acide 2-acrylamido-2-méthylpropane sulfonique n'est pas soluble dans le solvant acrylonitrile, en conséquence le produit de réaction est sous une forme de suspension de cristaux dans le solvant de réaction.

A titre d'exemple, les documents US 6,448,347 CN101066940 et CN 102351744 décrivent un procédé de fabrication de l'acide 2-acrylamido-2-méthylpropane sulfonique selon un mode continu.

L'acide 2-acrylamido-2-méthylpropane sulfonique est par la suite séparé de l'acrylonitrile, généralement par filtration et peut être purifié ultérieurement par plusieurs méthodes connues. En effet, une purification est nécessaire car un faible taux d'impuretés provenant de l'acide 2-acrylamido-2-méthylpropane sulfonique affecte fortement la polymérisation, et plus particulièrement le poids moléculaire et le taux d'insolubles dans l'eau.

Ainsi, dans le document WO2009/072480 qui porte sur un procédé de fabrication d'acide 2-acrylamido-2-méthylpropanesulfonique (ATBS) il est expliqué que les impuretés IBSA (l'acide 2-méthyl-2-propényl-1-sulfonique) et IBDSA (acide 2-méthylidène-1,3-propylènedisulfonique) affectent fortement la polymérisation au-delà d'une certaine concentration.

Il existe ainsi de nombreuses méthodes de purification. On peut citer à titre d'exemple le document US 6,331,647 qui décrit une méthode de purification de l'acide 2-acrylamido-2-méthylpropane sulfonique par recristallisation de son sel de sodium, l'eau étant le solvant de recristallisation. Toutefois, les sels de l'acide 2-acrylamido-2-méthylpropane sulfonique étant très solubles dans l'eau (>150g/l), le procédé de recristallisation est complexe et fastidieux pour un rendement qui est faible.

Le document US 4,337,215 décrit une méthode de purification de l'acide 2-acrylamido-2-méthylpropane sulfonique par recristallisation dans de l'acide acétique, par dissolution à chaud et cristallisation par rampe de refroidissement. Malgré la bonne pureté de l'acide 2-acrylamido-2-méthylpropane sulfonique obtenu, le procédé a un rendement limité, fait intervenir de multiples étapes de dissolution/refroidissement et requiert un traitement de l'acide acétique usagé pour le régénérer par distillation avant réutilisation ultérieure dans un nouveau batch de recristallisation de l'acide 2-acrylamido-2-méthylpropane sulfonique.

Le document US 2013/0137893 décrit une méthode de purification de l'acide 2-acrylamido-2-méthylpropane sulfonique par recristallisation de son sel de sodium dans un solvant anhydre. Le procédé est complexe en faisant appel à des solvants organiques et des bases anhydres qui sont difficiles à manipuler. De plus, ce document ne mentionne pas les impuretés créées par addition de Michael de la base anhydre sur la double liaison de l'acide 2-acrylamido-2-méthylpropane sulfonique.

La qualité de l'acide 2-acrylamido-2-méthylpropane sulfonique reste donc un problème majeur pour la fabrication de polymères comprenant ce monomère. Il faut donc un procédé simple pour la fabrication de l'acide 2-acrylamido-2-méthylpropane sulfonique de haute pureté, sans avoir recours à des étapes de purifications complexes et fastidieuses utilisant parfois des produits qui sont difficiles à manipuler.

Dans l'art antérieur, les impuretés et la qualité de l'isobutylène ne sont jamais prises en considération pour la fabrication de l'acide 2-acrylamido-2-méthylpropane sulfonique.

EXPOSE DE L'INVENTION

La présente invention a pour objet un procédé de fabrication de l'acide 2-acrylamido-2-méthylpropane sulfonique consistant à faire réagir entre eux l'acrylonitrile, l'acide sulfurique fumant et l'isobutylène. Dans ce procédé, l'isobutylène contient moins de 100 ppm de butadiène et moins de 100 ppm de butène.

La réaction mise en oeuvre dans le procédé de préparation de l'acide 2-acrylamido-2-méthylpropane sulfonique répond au schéma réactionnel ci-dessous, dans lequel l'acrylonitrile est présent en excès de manière à être à la fois le solvant de la réaction et un réactif. L'acrylonitrile est mis en contact avec de l'acide sulfurique et de l'isobutylène. L'acide sulfurique utilisé peut comprendre en outre des proportions variables de $SO_3$ libre.

Selon l'invention et de manière préférée l'acide sulfurique a une concentration comprise entre 105,6 % et 122,5%, correspondant à un oléum comprenant entre 25% et 100% de $SO_3$ libre. Plus préférentiellement l'acide sulfurique a une concentration comprise entre 105,6% et 113,5%, correspondant à un oléum ayant une concentration comprise entre 25% et 60% de $SO_3$ libre.

Le pourcentage de $SO_3$ libre se détermine par rapport au pourcentage de $H_2SO_4$ selon la formule suivante :

$$\%SO3 = \frac{(\%H2SO4-100)*M_{SO3}}{M_{H2O}}$$

Dans un mode de réalisation particulier, $SO_3$ et l'eau peuvent également être ajoutés séparément. Les formes alpha, beta ou gamma du $SO_3$ peuvent être utilisées indifféremment dans la présente invention.

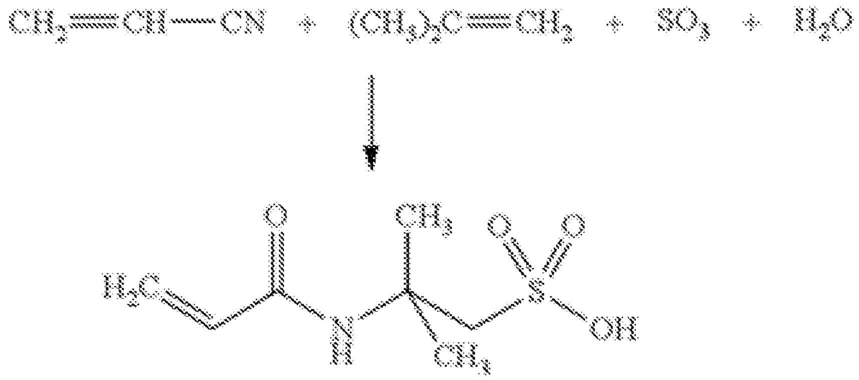

Schéma réactionnel de la préparation de l'acide 2-acrylamido-2-méthylpropane sulfonique

Le procédé peut être en fonctionnement continu, où l'acrylonitrile est d'abord versé puis mélangé avec de l'acide sulfurique tout en contrôlant la température dans une fourchette comprise entre -50°C et 20°C, préférentiellement entre -20°C et 0°C. Dans une seconde étape l'isobutylène est additionné au mélange précédant (acrylonitrile + acide sulfurique fumant), la température étant contrôlée entre -40 et 80°C, préférentiellement entre 30 et 50°C.

Le temps de contact entre l'isobutylène et le mélange précédant est avantageusement compris entre 10 secondes et 240 minutes, préférentiellement entre 10 et 120 minutes.

Les ratios molaires des réactifs sont typiquement déterminés pour maximiser la production d'acide 2-acrylamido-2-méthylpropane sulfonique. Le ratio $SO_3$ : isobutylène est généralement compris entre 0,2:1 et 2:1, préférentiellement entre 0,4:1 et 1,5:1, plus préférentiellement entre 0,7:1 et 1,2:1.

Le ratio acrylonitrile : isobutylène est généralement compris entre 1:1 et 20:1, préférentiellement entre 6:1 et 18:1, plus préférentiellement entre 12:1 et 16:1.

Lors de l'introduction de l'isobutylène, les cristaux de l'acide 2-acrylamido-2-méthylpropane sulfonique précipitent du milieu car ils ne sont pas solubles dans le solvant acrylonitrile, en conséquence le produit de réaction est sous une forme de suspension de cristaux dans le solvant de réaction.

L'isobutylène peut être introduit dans le milieu sous forme gazeuse pure ou diluée avec un gaz neutre, ou bien sous forme de gaz liquéfié, ou dissous dans un solvant. La réaction d'introduction de l'isobutylène peut donc être faite sous pression atmosphérique ou bien sous pression, par exemple jusqu'à 12 bars relatifs.

Les cristaux de l'acide 2-acrylamido-2-méthylpropane sulfonique sont ensuite séparés du solvant par une étape de séparation liquide/solide. A titre d'exemple et de manière non limitative, nous pouvons citer l'utilisation de centrifuge,

de décanteur, de filtre presse, de filtre à bande, de filtre à disques, ou de filtre à tambour rotatif.

Les cristaux de l'acide 2-acrylamido-2-méthylpropane sulfonique sont ensuite avantageusement séchés sous pression réduite ou atmosphérique.

Il est également possible d'effectuer le procédé de manière discontinue (en batch). En effet, le mode de production de l'acide 2-acrylamido-2-méthylpropane sulfonique acide (continu ou discontinu) ne conditionne en rien les bénéfices résultant de la présente invention. En d'autres termes, la présente invention s'utilise aussi bien avec un procédé de fabrication de l'acide 2-acrylamido-2-méthylpropane sulfonique en mode continu ou en mode batch.

L'acide 2-acrylamido-2-méthylpropane sulfonique acide peut également être préparé selon des procédés alternatifs comme par exemple celui décrit dans le document CN 102952052 qui revendique l'utilisation d'anhydride acétique en co-solvant de l'acrylonitrile afin de réduire et contrôler le taux d'eau dans la réaction pour s'affranchir l'utilisation d'acide sulfurique fumant.

L'isobutylène peut être fabriqué selon différentes méthodes connues de l'homme de métier. A titre d'exemples et de manière non limitative, l'isobutylène peut être obtenu par la déshydratation du tert-butanol ou de l'isobutanol, par la déshydrogénation de l'isobutane, par l'isomérisation du but-1-ène ou du but-2-ène, ou par le crackage du méthyl-tertiobutyléther (MTBE) etc...

Ces méthodes de production ont le défaut de produire des sous-produits tels que le butène et le butadiène. L'isolation de l'isobutylène requiert des étapes supplémentaires qui augmentent son coût de production.

Par butène, nous entendons toutes les formes non désirées pouvant être présentes dans l'isobutylène à l'issue de sa formation. Le butène comprend donc le but-1-ène ; le (Z)-but-2-ène, le (E)-but-2-ène, ces deux dernières formes étant des isomères.

Comme déjà indiqué, l'isobutylène peut être purifié afin de réduire le taux de butène et de butadiène. L'homme de métier connait les différentes méthodes de purifications qui existent. A titre d'exemple et de manière non limitative, nous pouvons citer le document US 2014/0051819, qui décrit une purification par absorption du mélange impur d'isobutylène par du tamis moléculaire.

D'autres méthodes de purification de l'isobutylène sont également décrites dans les documents US 3,479,416 et US 6,242,661.

Il a été découvert de manière surprenante par la Demanderesse que l'acide 2-acrylamido-2-méthylpropane sulfonique de haute pureté pouvait être obtenu en contrôlant les taux de butène et de butadiène contenus dans l'isobutylène utilisé comme réactif.

Aussi, la pureté de l'acide 2-acrylamido-2-méthylpropane sulfonique résultant du procédé selon l'invention est avantageusement supérieure à 99.5%.

Selon l'invention, l'isobutylène comprend moins de 100 ppm de butène, préférentiellement moins de 10ppm.

Selon l'invention, l'isobutylène comprend moins de 100 1000 ppm de butadiène, moins de 10ppm. Les ppm sont massiques. Ici, ils sont exprimés par rapport à la masse de l'isobutylène.

La quantité de butène et de butadiène est mesurée avantageusement par chromatographie en phase gazeuse.

Le procédé objet de l'invention peut également comprendre une étape de formation d'un sel de l'acide 2-acrylamido-2-méthylpropane sulfonique une fois celui-ci formé. Il s'agit en général d'une étape de neutralisation de la fonction acide $-SO_3H$ de l'acide 2-acrylamido-2-méthylpropane sulfonique. Cette étape peut consister à neutraliser une partie ou l'intégralité des fonctions acides. Elle permet notamment de former un sel de métal alcalin, un sel de métal alcalino-terreux ou un sel d'ammonium par exemple.

La pureté du sel d'acide 2-acrylamido-2-méthylpropane sulfonique résultant du procédé selon l'invention est également avantageusement supérieure à 99.5%.

La présente invention concerne également l'obtention d'un polymère comprenant au moins comme monomère acide 2-acrylamido-2-méthylpropane sulfonique, ou de ses sels, dans lequel l'acide 2-acrylamido-2-méthylpropane sulfonique est obtenu selon le procédé décrit précédemment.

Ce procédé de préparation d'un polymère comprend les étapes suivantes :

- préparation de l'acide 2-acrylamido-2-méthylpropane sulfonique par réaction entre eux de l'acrylonitrile, de l'acide sulfurique fumant et de l'isobutylène, l'isobutylène contenant moins de 100 ppm de butadiène et moins de 100 ppm de butène ;
- polymérisation de l'acide 2-acrylamido-2-méthylpropane sulfonique, et/ou de sa forme neutralisée, seul ou en combinaison avec d'autres monomères.

Selon un mode de réalisation particulier, l'acide 2-acrylamido-2-méthylpropane sulfonique, et/ou sa forme neutralisée, peut être polymérisé en combinaison avec au moins un monomère non ionique.

Selon un autre mode de réalisation particulier, l'acide 2-acrylamido-2-méthylpropane, et/ou sa forme neutralisée, sulfonique peut être polymérisé en combinaison avec au moins un monomère cationique.

Selon un autre mode de réalisation particulier, l'acide 2-acrylamido-2-méthylpropane sulfonique, et/ou sa forme

neutralisée, peut être polymérisé en combinaison avec au moins un monomère anionique.

Ainsi, ce procédé peut permettre l'obtention d'un polymère d'acide 2-acrylamido-2-méthylpropane sulfonique, et/ou sa forme neutralisée, et d'au moins : un monomère non ionique et/ou un monomère cationique et/ou un monomère anionique.

Ainsi, optionnellement, le polymère obtenu selon le procédé de l'invention peut comprendre au moins un monomère non ionique qui est avantageusement sélectionné dans le groupe comprenant l'acrylamide, le méthacrylamide, des esters d'alkyle hydroxyle d'acide acrylique, des esters d'alkyle hydroxyle d'acide méthacrylique, du N-vinyle pyrrolidone, du N-vinyle formamide et du polyéthylène glycol méthacrylate. Préférentiellement le monomère non ionique est l'acrylamide.

Optionnellement le polymère obtenu selon le procédé de l'invention peut comprendre au moins un monomère anionique qui est avantageusement sélectionné dans le groupe comprenant l'acide acrylique, l'acide méthacrylique, l'acide allyle sulfonique, ou des sels de ceux-ci. Préférentiellement, le monomère anionique est de l'acide acrylique ou un sel de celui-ci.

Optionnellement le polymère obtenu selon le procédé de l'invention peut comprendre au moins un monomère cationique qui est avantageusement sélectionné dans le groupe comprenant l'acrylate de diméthyl aminoéthyle (ADAME) quaternisé ou salifié, le méthacrylate de diméthyl aminoéthyle (MADAME) quaternisé ou salifié, le diallyldiméthylamine chloré (DADMAC), l'acrylamidopropyl triméthylamine chloré (APTAC) et le méthacrylamidopropyl triméthylamine chloré (MAPTAC).

Les polymères obtenus par le procédé peuvent être utilisés comme dispersants, coagulants, floculants ou agent épaississants. Ils sont hydrosolubles ou hydrogonflants.

Les polymères obtenus par le procédé peuvent être linéaires, branchés, réticulés, peignes.

Les polymères précédemment décrits sont utiles dans les domaines de la récupération assistée du pétrole et du gaz (drilling, fracturation hydraulique, EOR, drag réduction), du traitement de l'eau, de l'agriculture, du papier, des mines, de la cosmétique, de la détergence, du textile, de la construction.

## EXEMPLES

### Protocole de préparation de l'acide 2-acrylamido-2-méthylpropane sulfonique

Dans un réacteur agité de 2000ml ayant une double enveloppe, sont ajoutés 1525 grammes d'acrylonitrile et 117,2 grammes d'acide sulfurique fumant titrant 103% $H_2SO_4$ (13% oleum). Le mélange est agité et refroidi par la double enveloppe du réacteur qui est maintenue à - 20°C.

97 grammes d'isobutylène sont additionnés au mélange précèdent, à un débit de 1,6 grammes/minute.

La température du mélange est contrôlée à 45°C lors de l'introduction de l'isobutylène. Les cristaux de l'acide 2-acrylamido-2-méthylpropane sulfonique précipitent dans le mélange et le taux de solide est d'environ 20% en poids. Le mélange est filtré sur un filtre de type Buchner, et les cristaux blancs sont lavés 2 fois avec 300 grammes d'acrylonitrile vierge.

Les solides obtenus sont séchés sous vide à 50°C pendant deux heures.

La pureté de l'acide 2-acrylamido-2-méthylpropane sulfonique est mesurée par méthode HPLC (chromatographie en phase liquide à haute performance) selon les conditions suivantes :

- Colonne ODS-3 (GL Science trademark) ;
- Phase mobile : eau avec 0,03% acide trifluoroacétique / Acétonitrile (ratio massique 90/10) ;
- Débit phase mobile : 0,8m1/minute ;
- Longueur d'onde de détection : 200nm ;

Les mesures des quantités de butène et de butadiène ont été réalisées par chromatographie en phase gazeuse dans les conditions suivantes :

- Colonne Petrocol DH, 100m x 0.25 mm ID, 0.5μm film ;
- Four : de -50°C (10 min) à 75°C avec un gradient de 5°C/min ;
- Détecteur : FID ;

- Volume d'injection : 250µl ;
- Gaz vecteur : Hélium ;
- Ratio de division : 100 : 1.

Différentes puretés d'isobutylène et d'acide 2-acrylamido-2-méthylpropane sulfonique ont été testées selon le protocole ci-dessus.

Tableau 1 : pureté de l'isobutylène et de l'acide 2-acrylamido-2-méthylpropane sulfonique obtenu à partir de l'isobutylène

| | Pureté de l'isobutylène | | Pureté de l'acide 2-acrylamido-2-méthylpropane sulfonique (%massique) |
|---|---|---|---|
| | Quantité de butène (ppm) | Quantité de butadiène (ppm) | |
| Exemple 1 (invention) | 1 | 1 | 99,8 |
| Exemple 2 (invention) | 20 | 10 | 99,7 |
| Exemple 3 (invention) | 8 | 3 | 99,8 |
| Exemple 4 (invention) | 4 | 2 | 99,8 |
| Exemple 5 | 200 | 120 | 99,4 |
| Exemple 6 | 800 | 520 | 99,1% |
| Exemple 7 (contre-exemple) | 1250 | 1100 | 98,5% |

D'après les résultats, il apparait clairement que l'acide de 2-acrylamido-2-méthylpropane sulfonique est de meilleure pureté si les impuretés contenues dans l'isobutylène sont contrôlées à un taux inférieur à 1000ppm.
L'écart de pureté entre les exemples 1 à 6 et l'exemple 7 est significatif dans ce domaine de la production d'acide 2-acrylamido-2-méthylpropane sulfonique.

Protocole de polymérisation d'un copolymère d'acide 2-acrylamido-2-méthylpropane sulfonique et d'acrylamide.

Dans 60 grammes d'eau sont ajoutés 40 grammes d'acide 2-acrylamido-2-méthylpropane sulfonique obtenus selon les exemples 1 à 5. Une solution caustique à 50% en poids de NaOH est ajoutée de manière à contrôler le pH à 8. De l'eau est ensuite rajoutée afin d'ajuster la concentration du sel de sodium de l'acide 2-acrylamido-2-méthylpropane sulfonique à 35% en poids.
55,6gammes d'une solution aqueuse d'acrylamide à 40% en poids sont ajoutés, et une quantité supplémentaire d'eau est ajoutée de nouveau de manière à ajuster la quantité totale de monomères à 35% en poids.
700mg d'un initiateur azoïque, est ajouté en une fois au mélange précèdent.
730mg de persulfate de sodium ainsi que 700mg de sulfite de sodium sont ajoutés afin d'initier la polymérisation. La réaction est finie après 3 heures. Le gel obtenu est ensuite coupé, séché et tamisé.

Protocole de test de viscosité UL.

500mg de polymère sont ajoutés dans 490ml d'une solution d'eau déionisée. Après dissolution complète du polymère 29,25 grammes de NaCl sont ajoutés.
La viscosité est mesurée à l'aide d'un viscosimètre Brookfield digitale DVII+ sur une vitesse de rotation à 60 tours/minute à 25°C (module UL).

Protocole de test taux d'insolubles dans l'eau.

1 gramme de polymère est dissous dans 200ml d'une solution d'eau d'ionisée. Une toile métallique ayant une taille de pore de 200µm est séchée et pesée pour connaitre sa masse m1.
La solution de polymère est ensuite filtrée sur la toile métallique.
La toile métallique est ensuite séchée à l'étuve à 105°C pendant 4 heures, et est ensuite pesée pour connaitre

la masse m2.

Le taux d'insoluble est calculé selon la formule :

$$\% \text{ Insolubles} = 100 * (M2 - M1)$$

Les résultats de l'ensemble des polymères sont fournis dans le tableau 2. La performance des polymères a été évaluée en mesurant leur viscosité directement reliée au poids moléculaire que l'on souhaite tous deux comme le plus élevé. Elle a été également évaluée en mesurant le taux de solubles que l'on souhaite le plus faible possible.

Tableau 2 : Viscosité UL et taux d'insolubles des polymères obtenus à partir des différents l'acide 2-acrylamido-2-méthylpropane sulfonique

| | Origine de l'acide 2-acrylamido-2-méthylpropane sulfonique | Viscosité UL (cps) | Taux d'insolubles dans l'eau (%) |
|---|---|---|---|
| Polymère 1 | Exemple 1 (invention) | 5,2 | 0 |
| Polymère 2 | Exemple 2 (invention) | 4,8 | 0.01 |
| Polymère 3 | Exemple 3 (invention) | 5,0 | 0,008 |
| Polymère 4 | Exemple 4 (invention) | 5,1 | 0 |
| Polymère 5 | Exemple 5 (invention) | 4.6 | 0.1 |
| Polymère 6 | Exemple 6 (invention) | 4.4 | 1 |
| Polymère 7 | Exemple 7 (contre-exemple) | 4 | 3 |

Les polymères obtenus à partir d'acide 2-acrylamido-2-méthylpropane sulfonique selon l'invention ont des performances améliorées, à la fois en termes de viscosité UL et du taux d'insolubles.

Les exemples 8 et 9 suivants ont pour but de démontrer que le de fabrication d'ATBS selon l'invention permet d'obtenir des polymères ne nécessitant pas une étape de purification complexe et fastidieuse.

Exemple 8 : purification de l'acide 2-acrylamido-2-méthylpropane sulfonique obtenu selon l'exemple 7 selon le brevet US 4,337,215.

Dans un réacteur agité de 2000ml ayant une double enveloppe, sont ajoutés 1350 grammes d'acide acétique glacial ainsi que 150 grammes d'eau.

500 grammes du monomère obtenu à l'exemple 7 sont ajoutés dans le réacteur. La suspension ainsi obtenu est agitée.

Le mélange est chauffé à 90°C afin de dissoudre entièrement l'acide 2-acrylamido-2-méthylpropane sulfonique.

Après dissolution, le mélange est refroidi à une vitesse de 2°C/ 5 minutes, jusqu'à une température finale de 15°C. La suspension de cristaux est maintenue à 15°C durant 1 heure, et est ensuite filtré sur un filtre Buchner. Les cristaux obtenus sont lavés 2 fois avec 300 grammes d'acide acétique glacial.

Les cristaux sont séchés sous vide à une température de 60°C durant 3 heures.

Les cristaux secs ont un poids de 343 grammes, soit un rendement de 68%. La pureté est de 99,3%, soit une pureté correspondant à celle obtenue selon le procédé selon l'invention, notamment dans les exemples 5 et 6 pour lesquels aucune étape de purification de l'acide 2-acrylamido-2-méthylpropane sulfonique n'est faite.

Exemple 9 :

Le monomère purifié obtenu dans l'exemple 8 est copolymérisé selon le protocole décrit précédemment. Les résultats de viscosité UL ainsi que le taux d'insolubles sont mentionnés dans le tableau 3 ci-dessous.

Tableau 3 : Viscosité UL et taux d'insolubles du polymère obtenu à partir de l'acide 2-acrylamido-2-méthylpropane sulfonique de l'exemple 8.

| | Origine de l'acide 2-acrylamido-2-méthylpropane sulfonique | Viscosité UL (cps) | Taux d'insolubles dans l'eau (%) |
|---|---|---|---|
| Polymère 8 | Exemple 8 | 4.5 | 0.12 |

Le polymère 8 a des propriétés similaires au polymère 5, mais l'acide 2-acrylamido-2-méthylpropane sulfonique utilisé pour le produire a dû subir une étape de purification, ce qui n'a pas été le cas pour le polymère 5.

## Revendications

1. Procédé de fabrication de l'acide 2-acrylamido-2-méthylpropane sulfonique consistant à faire réagir entre eux de l'acrylonitrile, de l'acide sulfurique fumant et de l'isobutylène ***caractérisé* en ce que** l'isobutylène contient moins de 100ppm de butadiène et moins de 100ppm de butène.

2. Procédé selon la revendication 1, ***caractérisé* en ce qu'**il comprend une étape de formation d'un sel de l'acide 2-acrylamido-2-méthylpropane sulfonique une fois celui-ci formé.

3. Procédé de préparation d'un polymère comprenant les étapes suivantes :

   - préparation de l'acide 2-acrylamido-2-méthylpropane sulfonique selon la revendication 1 ;
   - polymérisation de l'acide 2-acrylamido-2-méthylpropane sulfonique seul ou en combinaison avec d'autres monomères.

4. Procédé selon la revendication 3, ***caractérisé* en ce que** l'acide 2-acrylamido-2-méthylpropane sulfonique, et/ou sa forme neutralisée, est polymérisé en combinaison avec au moins un monomère non ionique.

5. Procédé selon la revendication 3 ou 4, ***caractérisé* en ce que** l'acide 2-acrylamido-2-méthylpropane sulfonique, et/ou sa forme neutralisée, est polymérisé en combinaison avec au moins un monomère cationique.

6. Procédé selon l'une des revendications 3 à 5, ***caractérisé* en ce que** l'acide 2-acrylamido-2-méthylpropane sulfonique, et/ou sa forme neutralisée, est polymérisé en combinaison avec au moins un monomère anionique.

7. Procédé selon la revendication 1, ***caractérisé* en ce que** l'acide 2-acrylamido-2-méthylpropane sulfonique résultant de la réaction entre eux de l'acrylonitrile, de l'acide sulfurique fumant et de l'isobutylène présente une pureté supérieure à 99.5%.

## Patentansprüche

1. Verfahren zur Herstellung von 2-Acrylamido-2-Methylpropansulfonsäure, daraus bestehend, Acrylnitril, rauchende Schwefelsäure und Isobutylen miteinander reagieren zu lassen, **dadurch gekennzeichnet, dass** Isobutylen weniger als 100 ppm Butadien und weniger als 100 ppm Buten enthält.

2. Verfahren nach Anspruch 1, **dadurch gekennzeichnet, dass** es eine Etappe zur Bildung eines Salzes von 2-Acrylamido-2-Methylpropansulfonsäure umfasst, sobald Letzteres gebildet ist.

3. Verfahren zur Herstellung eines Polymers, umfassend die folgenden Schritte:

   - Vorbereitung von 2-Acrylamido-2-Methylpropansulfonsäure wie in Anspruch 1 definiert;
   - Polymerisierung von 2-Acrylamido-2-Methylpropansulfonsäure allein oder in Kombination mit anderen Monomeren.

4. Verfahren nach Anspruch 3, **dadurch gekennzeichnet, dass** 2-Acrylamido-2-Methylpropansulfonsäure und/oder ihre neutralisierte Form in Kombination mit mindestens einem nichtionischen Monomer polymerisiert wird.

5. Verfahren nach Anspruch 3 oder 4, **dadurch gekennzeichnet, dass** 2-Acrylamido-2-Methylpropansulfonsäure und/oder ihre neutralisierte Form in Kombination mit mindestens einem kationischen Monomer polymerisiert wird.

6. Verfahren nach einem der Ansprüche 3 bis 5, **dadurch gekennzeichnet, dass** 2-Acrylamido-2-Methylpropansulfonsäure und/oder ihre neutralisierte Form in Kombination mit mindestens einem anionischen Monomer polymerisiert wird.

7. Verfahren nach Anspruch 1, **dadurch gekennzeichnet, dass** die 2-Acrylamido-2-Methylpropansulfonsäure, die aus der Reaktion von Acrylnitril, rauchender Schwefelsäure und Isobutylen miteinander resultiert, eine Reinheit von mehr als 99,5% aufweist.

## Claims

1. A production process for 2-acrylamido-2-methylpropane sulfonic acid consisting of co-reacting acrylonitrile, fuming sulfuric acid and isobutylene, ***characterized* in that** the isobutylene contains less than 100 ppm of butadiene and less than 100 ppm of butene.

2. The process according to claim 1, ***characterized* in that** it comprises a step of formation of a salt of 2-acrylamido-2-methylpropane sulfonic acid once it has been formed.

3. A process for preparing a polymer comprising the following steps:

   - preparation of 2-acrylamido-2-methylpropane sulfonic acid according to claim 1;
   - polymerization of 2-acrylamido-2-methylpropane sulfonic acid alone or in combination with other monomers.

4. The process according to claim 3, ***characterized* in that** the 2-acrylamido-2-methylpropane sulfonic acid, and/or its neutralized form, is polymerized in combination with at least one nonionic monomer.

5. The process according to claim 3 or 4, ***characterized* in that** the 2-acrylamido-2-methylpropane sulfonic acid, and/or its neutralized form, is polymerized in combination with at least one cationic monomer.

6. The process according to one of claims 3 to 5, ***characterized* in that** the 2-acrylamido-2-methylpropane sulfonic acid, and/or its neutralized form, is polymerized in combination with at least one anionic monomer.

7. The process according to claim 1, ***characterized* in that** the 2-acrylamido-2-methylpropane sulfonic acid resulting from the co-reaction of acrylonitrile, fuming sulfuric acid and isobutylene presents purity greater than 99.5%.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- JP 2010270170 A
- US 6448347 B
- CN 101066940
- CN 102351744
- WO 2009072480 A
- US 6331647 B
- US 4337215 A
- US 20130137893 A
- CN 102952052
- US 20140051819 A
- US 3479416 A
- US 6242661 B